(19)

Europäisches
Patentamt
European
Patent Office
Office européen
des brevets

(11)  **EP 4 606 291 A1**

(12)  **EUROPEAN PATENT APPLICATION**

(43) Date of publication:
**27.08.2025 Bulletin 2025/35**

(21) Application number: **24159271.6**

(22) Date of filing: **23.02.2024**

(51) International Patent Classification (IPC):
***A61B 1/00*** (2006.01)   ***A61B 1/005*** (2006.01)

(52) Cooperative Patent Classification (CPC):
**A61B 1/000094; A61B 1/00006; A61B 1/000096;**
**A61B 1/0016; A61B 1/0051**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Medintech Inc.**
**Seoul 03100 (KR)**

(72) Inventors:
• **LEE, Chi Won**
**Namyangju-si, Gyeonggi-do (KR)**

• **KIM, Myung Joon**
**Gwacheon-si, Gyeonggi-do (KR)**
• **KIM, Tae Hyeon**
**Seoul (KR)**
• **CHO, Seok Ho**
**Seoul (KR)**
• **KIM, Gun O**
**Seoul (KR)**
• **KIM, Dong Hyeok**
**Seoul (KR)**

(74) Representative: **Gualeni, Nadia**
**Jacobacci & Partners S.p.A.**
**Piazza della Vittoria, 11**
**25122 Brescia (IT)**

(54)  **ENDOSCOPIC DEVICE AND CONTROL METHOD OF THE SAME**

(57)     Provided are an endoscopic device (100) for identifying a lesion based on a pre-trained model and tracking the identified lesion, and a method of controlling the endoscopic device. The method of controlling the endoscopic device includes obtaining an image inside a body from an image sensor (153a), identifying a lesion from the image based on a pre-trained model, and controlling a tip portion (153) of the endoscopic device to track the lesion.

FIG. 1

**Description**

[Technical Field]

The disclosure relates to an endoscopic device and a method of controlling the endoscopic device, and more particularly, to an endoscopic device for identifying a lesion based on a pre-trained model and tracking the identified lesions, and a method of controlling the endoscopic device.

[Background Art]

An endoscopic device is a medical instrument for inserting a scope into a human body to observe organs and perform a treatment or procedure as needed. Usually, a user using an endoscopic device simultaneously manipulates a scope and performs a medical operation of checking for abnormalities inside a human body. In such a situation where the user's concentration is dispersed, the user may miss a lesion that requires observation or treatment, depending on the user's skill level or fatigue.

In addition, when a lesion is discovered during investigation using an endoscopic device, in order to observe a discovered lesion or examine a tissue of the lesion, it is necessary to continuously manipulate a scope such that an image sensor faces the lesion. At this time, the user may wrongly manipulate the scope while focusing on a medical treatment. In this case, the scope is shaken and the lesion may escape out of the screen, and thus, the user needs to find the lesion again.

The above-mentioned background art is technical information possessed by the inventor for the derivation of the disclosure or acquired during the derivation of the disclosure, and cannot be considered as a known technique disclosed to the general public prior to the filing of the disclosure.

[Disclosure of Invention]

[Technical Problem]

Provided are an endoscopic device for identifying a lesion based on a pre-trained model and tracking the identified lesion, and a method of controlling the endoscopic device.

However, the above objective is an example, and the objectives of the disclosure are not limited thereto. Other objectives than the above may be clearly understood by those of skill in the art from the present specification and the accompanying drawings.

[Solution to Problem]

According to an aspect of the disclosure, a method of controlling an endoscopic device includes obtaining an image inside a body from an image sensor, identifying a lesion from the image based on a pre-trained model, and controlling a tip portion of the endoscopic device to track the lesion.

In the present embodiment, the angle of view of the image sensor may include a first area, and the controlling of the tip portion may include controlling the tip portion such that the lesion is located within the first area.

In the present embodiment, the first area may include the center of the angle of view.

In the present embodiment, the identifying of the lesion may include calculating on-image lesion position information indicating a position of the lesion on the image.

In the present embodiment, the controlling of the tip portion may include calculating a first value defined as a ratio of a difference in a position on the image to a change in an angle of the tip portion.

In the present embodiment, the controlling of the tip portion may include obtaining a first image when the tip portion is at a first angle and obtaining a second image when the tip portion is at a second angle, and measuring a difference between the first angle and the second angle, and a position difference between the first image and the second image.

In the present embodiment, the controlling of the tip portion may further include calculating a total movement angle of the tip portion based on the first value.

According to another aspect of the disclosure, a method of controlling an endoscopic device includes obtaining an image inside a body from an image sensor, identifying a plurality of lesions from the image based on a pre-trained model, and controlling a tip portion of the endoscopic device to track one target lesion from among the plurality of lesions.

In the present embodiment, the method may further include outputting on-image lesion position information for the plurality of identified lesions, and selecting the target lesion from among the plurality of lesions, in response to a user input on a manipulation unit.

In the present embodiment, the user input may include at least one of a single input, an input for a threshold time or longer, and a double input within the threshold time.

According to another aspect of the disclosure, an endoscopic device includes a tip portion including an image sensor, and a control unit configured to obtain an image inside a body from the image sensor, identify a lesion from the image based on a pre-trained model, and control the tip portion to track the lesion.

In the present embodiment, the angle of view of the image sensor may include a first area, and the control unit may be further configured to control the tip portion such that the lesion is located within the first area.

In the present embodiment, the first area may include the center of the angle of view.

In the present embodiment, the control unit may be further configured to determine on-image lesion position information indicating a position of the lesion on the image.

In the present embodiment, the control unit may be further configured to calculate a first value defined as a ratio of a difference in a position on the image to a change in an angle of the tip portion.

In the present embodiment, the control unit may be further configured to obtain a first image when the tip portion is at a first angle and obtain a second image when the tip portion is at a second angle, and measure a difference between the first angle and the second angle, and a position difference between the first image and the second image.

In the present embodiment, the control unit may be further configured to calculate a total movement angle of the tip portion based on the first value.

According to another aspect of the disclosure, an endoscopic device includes a tip portion including an image sensor, and a control unit configured to obtain an image inside a body from the image sensor, identify a plurality of lesions from the image based on a pre-trained model, and control the tip portion to track one target lesion from among the plurality of lesions.

In the present embodiment, the endoscopic device may further include a display unit configured to output the image, and a manipulation unit configured to receive an input from a user, the control unit may be further configured to output, on the display unit, on-image lesion position information for the identified plurality of lesions, and the target lesion is selected from among the plurality of lesions, in response to a user input on the manipulation unit.

In the present embodiment, the user input may include at least one of a single input, an input for a threshold time or longer, and a double input within the threshold time.

Other aspects, features, and advantages than those described above will become clear from the following detailed description, claims, and drawings for carrying out the disclosure.

[Advantageous Effects of Invention]

An endoscopic device and a method of controlling the endoscopic device according to an embodiment may detect a lesion inside a body without being affected by a user's skill level and fatigue, by using a pre-trained model to identify the lesion.

The endoscopic device and the method of controlling the endoscopic device according to an embodiment cause a tip portion to track an automatically identified lesion, enabling even a low-skilled user to track and observe a lesion without difficulty and to focus on observation, treatment, and procedures of an identified lesion without having to pay attention to manipulation of a scope, thereby improving the quality of treatment.

The effects of the disclosure are not limited to those described above, and other effects not described herein may be clearly understood by one of skill in the art from the present specification and the accompanying drawings.

[Brief Description of Drawings]

FIG. 1 is a diagram illustrating an endoscopic device according to an embodiment.

FIG. 2 is a diagram illustrating an example in which a pre-trained model identifies a lesion from images inside a body, according to an embodiment.

FIG. 3 is a diagram illustrating an image output on a display unit when a lesion is identified.

FIG. 4 is a diagram illustrating in detail a method of calculating a first value based on a position difference on images according to a difference in an angle of a tip portion.

FIG. 6 is a diagram illustrating an image output on a display unit when a lesion is located in a first area.

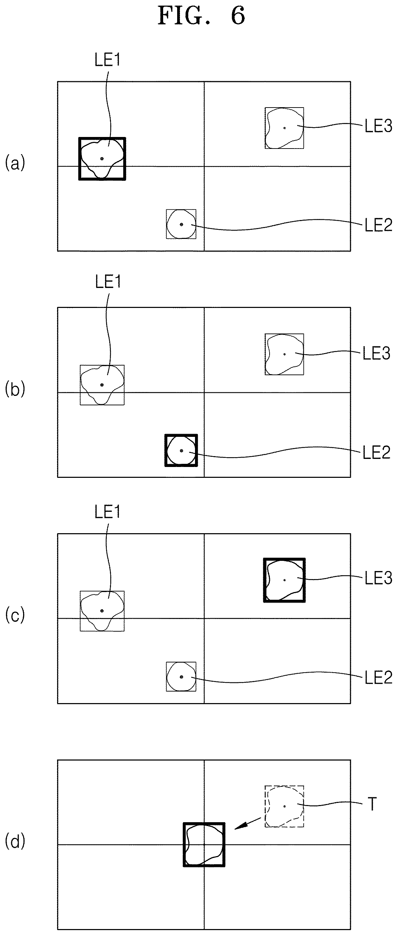

FIG. 6 is a diagram illustrating an example in which a target lesion is selected from a plurality of lesions in response to a user input on a manipulation unit.

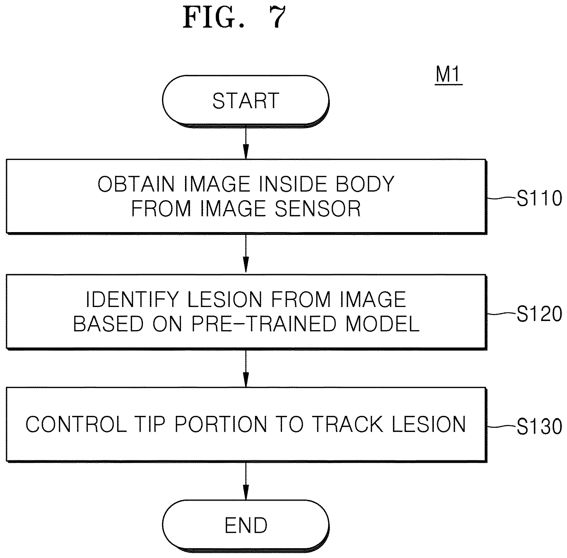

FIG. 7 is a flowchart of a method of controlling an endoscopic device, according to an embodiment.

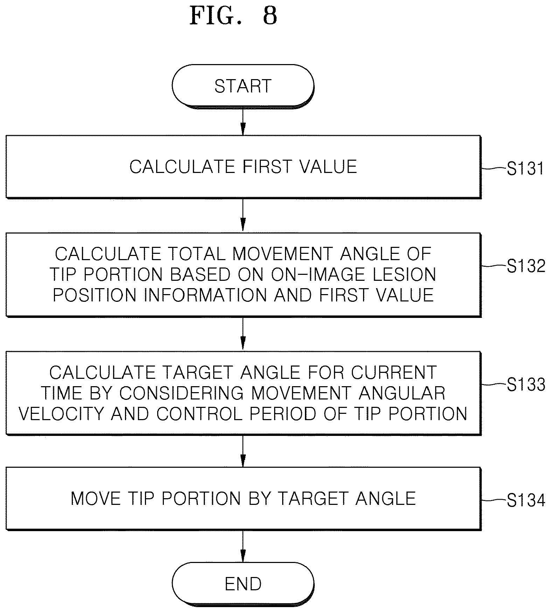

FIG. 8 is a flowchart of sub-operations of controlling a tip portion, according to an embodiment.

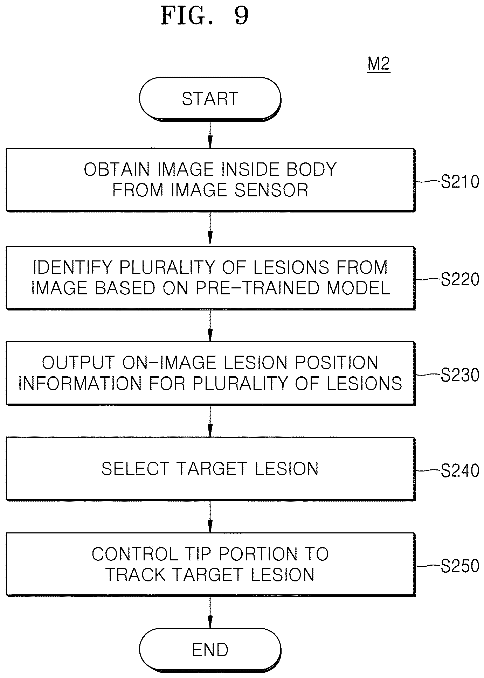

FIG. 9 is a flowchart of a method of controlling an endoscopic device, according to another embodiment.

[Mode for Invention]

Terms used herein are for describing particular embodiments and are not intended to limit the scope of other embodiments. The singular expression may also include the plural meaning as long as it is not inconsistent with the context. All the terms used herein, including technical and scientific terms, may have the same meanings as those generally understood by those of skill in the art. The terms as defined in a general dictionary may be interpreted as the same meanings as the contextual meanings of a related technology, and are not interpreted as ideal or excessively formal meanings unless defined clearly in the disclosure. In some cases, even the terms defined in the disclosure should not be interpreted to exclude the embodiments of the disclosure.

Hereinafter, various embodiments will be described in detail with reference to the accompanying drawings for those of skill in the art to be able to implement the embodiments without any difficulty. The disclosure may, however, be embodied in many different forms and is not limited to the embodiments set forth herein. When it is determined that, in describing embodiments disclosed herein, detailed description on function or configuration of related known technologies may unnecessarily confuse the gist of the disclosure, the detailed description will be omitted. The same or similar components will be assigned the same reference numeral and redundant descriptions thereof will be omitted.

The term "... unit" or "...er (or)" as used herein refers to a component configured to perform a particular function that is performed by software or hardware such as a field-programmable gate array (FPGA) or an application-specific integrated circuit (ASIC). However, the term "... unit" or "...er (or)" is not limited to being performed by software or hardware. The term "... unit" or "...er (or)" may exist in the form of data stored in an addressable storage medium, or may be implemented by instructions such that one or more processors execute a particular function.

The software may include a computer program, code, instructions, or a combination of one or more thereof, and may configure the processor to operate as desired or may independently or collectively instruct the processor. Software and/or data may be embodied permanently or temporarily in any type of machine, component, physical or virtual equipment, computer storage medium or device, or in a propagated signal wave capable of providing instructions or data to or being interpreted by the processor. The software may be distributed on networked computer systems and stored or executed in a distributed manner. The software and data may be stored in one or more computer-readable recording media. The software may be read into a main memory from another computer-readable medium, such as a data storage device, or from another device via a communication interface. Software instructions stored in the main memory may cause a processor to perform processes or operations that will be described in detail below. Alternatively, hardwired circuitry may be used instead of or in combination with software instructions to execute processes consistent with the principles of the disclosure. Thus, embodiments consistent with the principles of the disclosure are not limited to any particular combination of hardware circuitry and software.

Terms used herein are merely used to describe a particular embodiment, and are not intended to limit the disclosure. Singular forms are intended to include plural forms as well, unless the context clearly indicates otherwise. As used herein, terms such as "comprises," "includes," or "has" specify the presence of stated features, numbers, stages, operations, components, parts, or a combination thereof, but do not preclude the presence or addition of one or more other features, numbers, stages, operations, components, parts, or a combination thereof. Terms such as "first" or "second" may be used to describe various elements, but the elements should not be limited by the terms. These terms are used only to distinguish one component from another.

The term 'learning model' as used herein may encompass any type of algorithm or methodology used to learn or understand a particular pattern or structure from data. That is, the term 'learning model' may encompass not only machine learning models such as regression models, decision trees, random forests, support vector machines, K-nearest neighbors, Naive Bayes, or clustering algorithms, but also deep learning models such as neural networks, convolutional neural networks, recurrent neural networks, transformer-based neural networks, generative adversarial networks (GANs), or autoencoders. The term 'learning model' may refer to a set of trained parameters or weights that are used to predict or classify an output for a particular input, and such a model may be trained by performing methods such as supervised learning, unsupervised learning, semi-supervised learning, or reinforcement learning. In addition, it may include not only a single model but also various learning methods and structures, such as ensemble models, multimodal models, and models through transfer learning. Such learning models may be pre-trained on a computer device separate from the computer device that predicts an output for an input, and used by another computer device.

A learning model according to an embodiment may include models for object detection and position estimation.

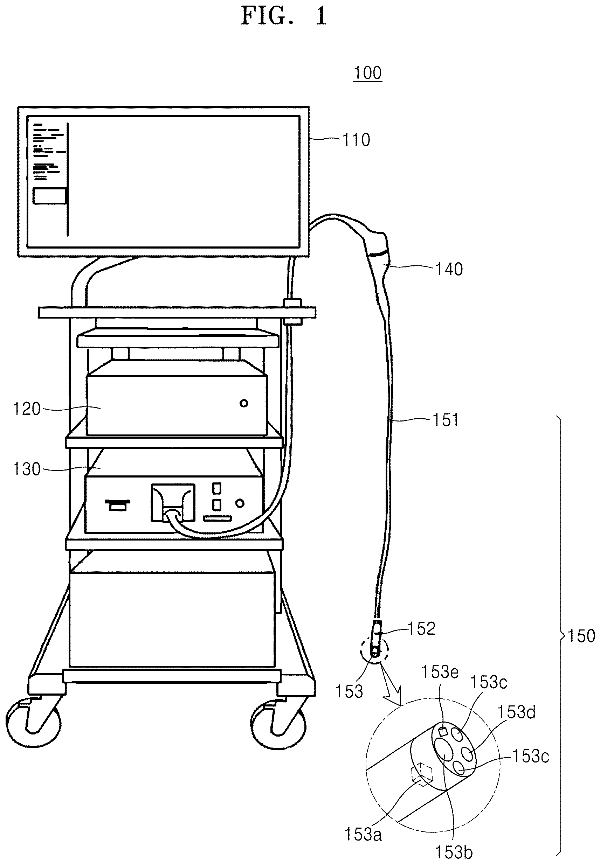

Hereinafter, an endoscopic device and a method of controlling the endoscopic device according to embodiments will be described with reference to FIGS. 1 to 9.

FIG. 1 is a diagram illustrating an endoscopic device 100 according to an embodiment.

The endoscopic device 100 is a medical instrument for inserting a scope into a human body to observe organs and perform a treatment or procedure as needed. Referring to FIG. 1, the endoscopic device 100 may include a display unit 110, a control unit 120, a driving unit 130, a manipulation unit 140, and a scope 150.

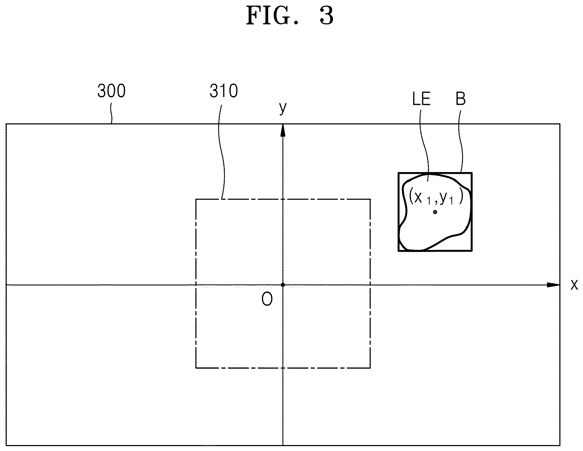

The display unit 110 may output an image. In other words, the display unit 110 may output an internal body image obtained from an image sensor 153a to be described below. The output internal body image may include x-axis coordinates and y-axis coordinates (see FIG. 3).

The display unit 110 may include a display module capable of outputting visualized information or implementing a touch screen, such as a liquid-crystal display (LCD), a thin-film transistor-LCD (TFT-LCD), an organic light-emitting diode (OLED) display, a flexible display, or a three-dimensional (3D) display.

The display unit 110 may output on-image lesion position information P, which will be described below.

The control unit 120 may control the overall operation of the endoscopic device 100. The control unit 120 may include all types of components capable of processing data. In an embodiment, the control unit 120 may include a hardware-embedded data processing device having a physically structured circuitry to perform functions represented by code or instructions included in a program. The hardware-embedded data processing device may include a processing device such as a microprocessor, a central processing unit (CPU), a processor core, a multiprocessor, an application-specific integrated circuit (ASIC), or a field-programmable gate array (FPGA).

The control unit 120 may control the angle of a tip portion 153 through the driving unit 130, which will be described below. The control unit 120 will be described in detail below.

The driving unit 130 may provide power necessary for the scope 150, which will be described below, to be inserted into a body or to move inside the body. For example, the driving unit 130 may include a plurality of motors connected to wires within the scope 150, and a tension control unit configured to adjust the tension of the wires.

The manipulation unit 140 may input a command of a user. The manipulation unit 140 may include a plurality of buttons that provide various functions to control the angle of the tip portion 153, which will be described below, and to perform various procedures inside the body.

In an embodiment, commands of the user may include a command to, when a plurality of lesions LE are identified in an image, select a target lesion T from among the plurality of lesions LE. The target lesion T refers to the lesion LE that the tip portion 153 is to track. In an embodiment, the manipulation unit 140 may include a selection button (not shown). A detailed example of the above will be described below.

The scope 150 may be directly inserted into the body. In detail, the scope 150 may include an insertion unit 151, a bending portion 152, and the tip portion 153.

The insertion unit 151 may serve to insert the tip portion 153, which will be described below, to an arbitrary position inside the body that is subject to observation and treatment. The insertion unit 151 may be connected to one end of the manipulation unit 140.

The bending portion 152 may be connected to one end of the insertion unit 151. The bending portion 152 may serve to change the angle of the tip portion 153, which will be described below. The bending portion 152 may be flexibly bent. As the bending portion 152 is bent, the angle of the tip portion 153 may be changed. The bending portion 152 may be connected to the driving unit 130 to receive a force necessary to change the angle of the tip portion 153. The degree or direction of bending of the bending portion 152 may be determined by the driving unit 130.

The tip portion 153 may be connected to one end of the bending portion 152. The tip portion 153 may photograph the inside of the body, and perform a treatment or a procedure as needed. The tip portion 153 may include the image sensor 153a, a lens 153b, a light 153c, a working channel 153d, and an air/water channel 153e.

The image sensor 153a may serve to obtain an image inside the body. The image inside the body may include a video image consisting of a plurality of consecutive frames. The image inside the body may be output through the display unit 110.

The angle of view of the image sensor 153a may include a first area. The first area may include the center of the angle of view. In an image 300 inside the body, a portion 310 corresponding to the first area may include the center of the image 300 (see FIG. 3). Thus, when a target is located in the first area, the target may be located close to the center of the image output through the display unit 110.

The lens 153b may serve as a passage through which light reflected inside the body enters the image sensor 153a. The light 153c may emit light toward the inside of the body such that an image inside the body may be obtained by using the image sensor 153a. The number of lights 153c is not particularly limited. A tool for treating and processing the lesion LE may be inserted into the working channel 153d. Air or washing water may be supplied through the air/water channel 153e.

Hereinafter, the control unit 120 will be described in detail.

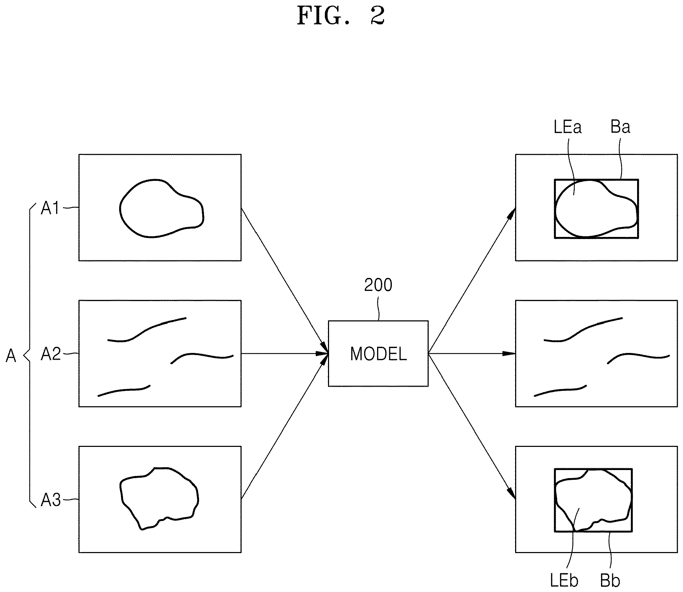

FIG. 2 is a diagram illustrating an example in which a pre-trained model 200 identifies the lesion LE from images A inside a body, according to an embodiment. FIG. 3 is a diagram illustrating the image 300 output on the display unit 110 when the lesion LE is identified.

The control unit 120 may include a processor configured to perform various calculations or operations to be described below. The processor may read a computer program to perform data processing for machine learning. The processor may perform computational processes such as processing input data for machine learning, extracting features for machine learning, or calculating errors based on backpropagation. The processor for performing such data processing may include a central processing unit (CPU), a general-purpose graphics processing unit (GPGPU), a tensor processing unit (TPU), an ASIC, an FPGA, or the like. However, this is only an example, and the type of the processor may be configured in various ways that may be understood by those of skill in the art based on the description.

The control unit 120 may obtain an image inside the body from the image sensor 153a, identify the lesion LE from the image based on the pre-trained model 200, and control the tip portion 153 to track the lesion LE.

The control unit 120 may train the model in advance. The model may be trained to receive an image inside a body, identify a lesion LE from the image inside the body, and determine on-image lesion position information P. The model may receive images inside a body as training data. In addition, the model may receive label data corresponding to each image inside the body, with the position of a lesion indicated as a bounding box.

The model may include a network structure such as a fully convolutional network (FCN), a conditional adversarial network (CAN), a recurrent neural network (RNN), or a matching cost-CNN (MC-CNN).

Referring to FIG. 2, the control unit 120 may identify the lesion LE from the images A based on the pre-trained model 200. The control unit 120 may input, into the pre-trained model 200, the images A inside the body obtained from the image sensor 153a. The pre-trained model 200 having received the input of the images A inside the body may identify the lesion LE and determine and output the on-image lesion position information P. The on-image lesion position information P may be information indicating the position of the lesion LE on the image output on the display unit 110. In an embodiment, as illustrated in FIG. 2, the on-image lesion position information P may include a bounding box B surrounding the lesion LE.

For example, as illustrated in FIG. 2, when an image A1 inside the body is input to the pre-trained model 200, the pre-trained model 200 may identify a lesion LEa included in the image A1 inside the body, and output a bounding box Ba surrounding the lesion LEa. When an image A2 inside the body is input to the pre-trained model 200, the image A2 inside the body does not have a lesion, and thus, the pre-trained model 200 does not output a bounding box. When an image A3 inside the body is input to the pre-trained model 200, the pre-trained model 200 may identify a lesion LEb included in the image A3 inside the body, and output a bounding box Bb surrounding the lesion LEb.

In an embodiment, the on-image lesion position information P may include on-image coordinate information for four line segments of the bounding box B. That is, the pre-trained model 200 may determine the on-image coordinate information for the four line segments of the bounding box B.

In an embodiment, the coordinates of the center of the bounding box B may be calculated from the on-image coordinate information for the four line segments of the bounding box B. Referring to FIG. 3, coordinates $(x_1, y_1)$ of the center of the bounding box B of the lesion LE identified by the pre-trained model 200 in the image 300 inside the body output on the display unit 110 may be calculated from the on-image coordinate information for the four line segments of the bounding box B. The coordinates $(x_1, y_1)$ of the center of the bounding box B may be referred to as the coordinates of the center of the identified lesion LE.

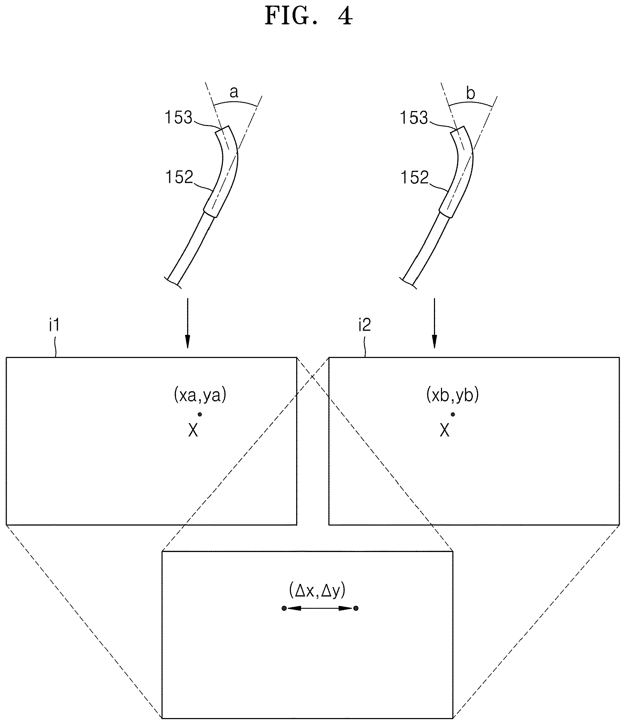

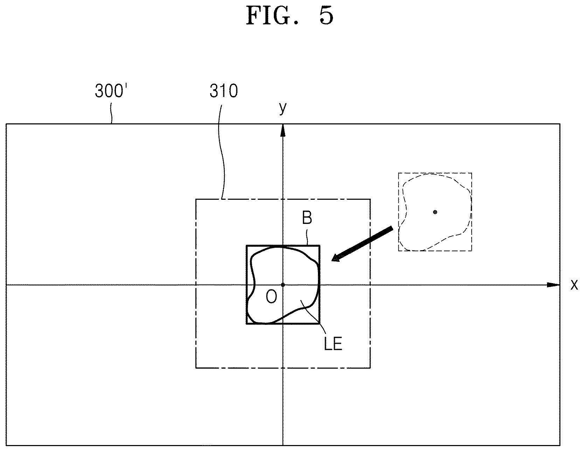

FIG. 4 is a diagram illustrating in detail a method of calculating a first value K based on a position difference on images according to a difference in an angle of the tip portion 153. FIG. 5 is a diagram illustrating an image 300' output on the display unit 110 when the lesion LE is located in the first area.

The control unit 120 may control the tip portion 153 to track the lesion LE. In detail, the control unit 120 may control the tip portion 153 such that the lesion LE is located within the first area. Accordingly, the identified lesion LE may be located close to the center of the image 300' output through the display unit 110. As a result, the identified lesion LE may be continuously output on the display unit 110.

The control unit 120 may calculate a first value K, which is the ratio of the difference in position on images to the change in the angle of the tip portion 153, calculate a total movement angle TMA of the tip portion based on the on-image lesion position information P and the first value K, calculate a target angle ag for the current time by considering a movement angular velocity as and a control period cp of the tip portion 153, and move the tip portion 153 by the target angle ag for the current time.

The control unit 120 may calculate the first value K, which is the ratio of the difference in position on images to the change in the angle of the tip portion 153. The first value K is a value necessary to calculate the total movement angle TMA of the tip portion 153, which will be described below.

In an embodiment, the control unit 120 may obtain a first image i1 when the tip portion 153 is at a first angle a, and a second image i2 when the tip portion 153 is at a second angle b, and measure a difference $\Delta\theta$ between the first angle a and the second angle b, and a position difference $\Delta X$ between the first image i1 and the second image i2. The first angle a and the second angle b are two different arbitrary angles for calculating the first value K, and may refer to angles between the tip portion 153 and the insertion unit 151, which are formed as the bending portion 152 is bent.

Referring to FIG. 4, the control unit 120 may obtain the first image i1 with the image sensor 153a when the tip portion 153 is caused to be at the first angle a by manipulating the bending portion 152. Here, the coordinates of an arbitrary point X inside the body on the image may be (xa, ya). The method of designating the arbitrary point X is not particularly limited. In an embodiment, the arbitrary point X may be the position of a blood vessel having a particular shape inside the body found on the image.

Again, the control unit 120 may obtain the second image i2 with the image sensor 153a when the tip portion 153 is caused to be at the second angle b by manipulating the bending portion 152. Here, the coordinates of an arbitrary point X inside the body on the image may be (xb, yb).

The coordinate difference between the first image i1 and the second image i2 may be expressed as ($\Delta$x, $\Delta$y), which is the difference between the coordinates of the arbitrary point X on the images. Here, $\Delta$x = xb - xa, and $\Delta$y = yb - ya. By using this, the position difference $\Delta$X between the first image i1 and the second image i2 is expressed as [Equation 1] below.

[Equation 1]

$$\Delta X = \sqrt{(\Delta x)^2 + (\Delta y)^2}$$

By applying the difference $\Delta\theta$ between the first angle a and the second angle b and the position difference $\Delta$X between the first image i1 and the second image i2 to [Equation 2] below, the first value K may be calculated.

[Equation 2]

$$K = \frac{\Delta X}{\Delta \theta}$$

The control unit 120 may calculate the total movement angle TMA of the tip portion based on the on-image lesion position information P and the first value K. The total movement angle TMA refers to a total angle by which the tip portion 153 needs to move to move the coordinates $(x_1, y_1)$ of the center of the lesion LE to the center coordinates (0, 0) on the image. Referring to FIG. 3, a length Q from the center of the lesion LE to the center of the image is as shown in [Equation 3] below.

[Equation 3]

$$Q = \sqrt{(x_1)^2 + (y_1)^2}$$

The total movement angle TMA may be calculated by applying the length Q from the center of the lesion LE to the center of the image, and the calculated first value K to [Equation 4] below.

[Equation 4]

$$(TMA) = \frac{Q}{K}$$

The control unit 120 may calculate the target angle ag for the current time by considering the movement angular velocity as and the control period cp of the tip portion 153. The movement angular velocity as and the control period cp of the tip portion 153 may be set in advance.

In an embodiment, the target angle ag for the current time may be calculated through a polynomial trajectory. As a specific example, the tip portion 153 may be set to move by the total movement angle TMA at a constant velocity. The preset movement angular velocity as of the tip portion 153 may be 30°/s, and the preset control period cp may be 2 ms. In this case, the target angle ag for the current time may be 30°/s × 2ms = 0.2 °. However, this is only an example for description and the disclosure is not limited thereto.

In another embodiment, the target angle ag for the current time may be calculated through a Bezier curve trajectory. That is, the method of calculating the target angle ag for the current time is not limited to the polynomial trajectory. In other words, the target angle ag for the current time may be calculated through various trajectories that may occur to one of skill in the art.

The control unit 120 may move the tip portion 153 by the target angle ag for the current time. The control unit 120 may calculate a force necessary to move the tip portion 153 by the target angle ag for the current time, by considering the dynamic characteristics of the bending portion 152. The control unit 120 may deliver information about the calculated force to the driving unit 130 to move the tip portion 153 by the target angle ag for the current time.

As illustrated in FIG. 5, the control unit 120 may repeatedly perform the process of identifying the lesion, calculating the first value K, and controlling the tip portion 153, until the lesion LE is located in the first area. Accordingly, the tip portion 153 may track the lesion LE, and the lesion LE may be continuously output on the display unit 110.

FIG. 6 is a diagram illustrating an example in which a target lesion T is selected from a plurality of lesions LE in response to a user input on the manipulation unit 140.

The control unit 120 may identify a plurality of lesions LE from an image, based on the pre-trained model 200. For example, referring to FIG. 6, a first lesion LE1, a second lesion LE2, and a third lesion LE3 may be identified in an image obtained from the image sensor 153a. The pre-trained model 200 may output bounding boxes surrounding the first lesion LE1, the second lesion LE2, and the third lesion LE3, respectively.

In response to a user input on the manipulation unit 140, the target lesion T may be selected from among the plurality of lesions LE. In detail, in response to a user input, a lesion LE designated from among the plurality of lesions LE may be changed, and the designated lesion LE may be selected as the target lesion T. As described above, the user may select the target lesion T from among the plurality of lesions LE by using the selection button (not shown) provided on the manipulation unit 140.

In an embodiment, the user input may include at least one of a single input, an input for a threshold time or longer, and a double input within the threshold time. For example, the single input may be a command to change a lesion LE designated from among the plurality of lesions LE. In addition, the double input within the threshold time may be a command to select the designated lesion LE as the target lesion T. However, this is only an example and the disclosure is not limited thereto.

In an embodiment, as illustrated in FIG. 6, the designated lesion LE may be specified by a bounding box having a different thickness. A detailed description with the above example is as follows. When the user presses the selection button once, the designated lesion LE may be changed from the first lesion LE1 to the second lesion LE2. When the user presses the selection button one more time, the designated lesion LE may change from the second lesion LE2 to the third lesion LE3. At this time, when the user presses the selection button twice within the threshold time, the designated third lesion LE3 may be selected as the target lesion T.

The practicality of the endoscopic device 100 may be improved by, when a plurality of lesions LE are identified in one image, tracking the target lesion T selected from among the plurality of lesions LE.

FIG. 7 is a flowchart of a method M1 of controlling an endoscopic device, according to an embodiment.

The method M1 of controlling an endoscopic device is a method of controlling the endoscopic device 100 including identifying a lesion LE in an image inside a body obtained from the endoscopic device 100, and causing the tip portion 153 to track the identified lesion LE such that the lesion LE is continuously output on the display unit 110.

Referring to FIG. 7, the method M1 of controlling an endoscopic device may include obtaining an image A inside a body from the image sensor 153a (S110), identifying a lesion LE from the image A based on the pre-trained model 200 (S120), and controlling the tip portion 153 to track the lesion LE (S130).

The endoscopic device 100 may obtain the image A inside the body from the image sensor 153a (S110). By inserting the scope 150 into the body, an image inside the body may be obtained through the image sensor 153a provided at the tip portion 153. The image inside the body may include a video image consisting of a plurality of consecutive frames. The image inside the body may be output through the display unit 110.

Referring to FIG. 2, the endoscopic device 100 may identify the lesion LE from the image A based on the pre-trained model 200 (S120). The endoscopic device 100 may input the image A inside the body obtained from the image sensor 153a, into the pre-trained model 200. The pre-trained model 200 having received the input of the image A inside the body may identify the lesion LE and determine and output on-image lesion position information P. The on-image lesion position information P may be information indicating the position of the lesion LE on the image output on the display unit 110. In an embodiment, as illustrated in FIG. 2, the on-image lesion position information P may include the bounding box B surrounding the lesion LE.

For example, as illustrated in FIG. 2, when the image A1 inside the body is input to the pre-trained model 200, the pre-trained model 200 may identify the lesion LEa included in the image A1 inside the body, and output the bounding box Ba surrounding the lesion LEa. When the image A2 inside the body is input to the pre-trained model 200, the image A2 inside the body does not have a lesion, and thus, the pre-trained model 200 does not output a bounding box. When the image A3 inside the body is input to the pre-trained model 200, the pre-trained model 200 may identify the lesion LEb included in the image A3 inside the body, and output the bounding box Bb surrounding the lesion LEb.

The model may be trained to receive an image inside a body, identify a lesion LE from the image inside the body, and determine on-image lesion position information P. The model may receive images inside a body as training data. In addition, the model may receive label data corresponding to each image inside the body, with the position of a lesion indicated as a bounding box.

The model may include a network structure such as an FCN, a CAN, an RNN, or an MC-CNN.

In an embodiment, the on-image lesion position information P may include on-image coordinate information for four line segments of the bounding box B. That is, the pre-trained model 200 may determine the on-image coordinate information for the four line segments of the bounding box B.

In an embodiment, the coordinates of the center of the bounding box B may be calculated from the on-image coordinate information for the four line segments of the bounding box B. Referring to FIG. 3, the coordinates $(x_1, y_1)$ of the center of the bounding box B of the lesion LE identified by the pre-trained model 200 in the image 300 inside the body output on the display unit 110 may be calculated from the on-image coordinate information for the four line segments of the bounding box B. The coordinates $(x_1, y_1)$ of the center of the bounding box B may be referred to as the coordinates of the center of the identified lesion LE.

Meanwhile, the angle of view of the image sensor 153a may include the first area. The first area may include the center of the angle of view. Referring to FIG. 3, the portion 310 corresponding to the first area in the image 300 inside the body may include the center of the image 300. Thus, when a target is located in the first area, the target may be located close to the center of the image output through the display unit 110.

FIG. 8 is a flowchart of sub-operations of the controlling of the tip portion (S130), according to an embodiment.

The endoscopic device 100 may control the tip portion 153 to track the lesion LE (S130). The controlling of the tip portion (S130) may include controlling the tip portion 153 such that the lesion LE is located within the first area. Accordingly, the identified lesion LE may be located close to the center of the image output through the display unit 110. As a result, the identified lesion LE may be continuously output on the display unit 110.

Referring to FIG. 8, the controlling of the tip portion (S130) may include calculating a first value K that is the ratio of the difference in position on images to the change in the angle of the tip portion 153 (S131), calculating a total movement angle TMA of the tip portion based on the on-image lesion position information P and the first value K (S132), calculating a target angle ag for the current time by considering a movement angular velocity as and a control period cp of the tip portion 153 (S133), and moving the tip portion 153 by the target angle ag for the current time (S134).

The endoscopic device 100 may calculate the first value K, which is the ratio of the difference in position on images to the change in the angle of the tip portion 153 (S131). The first value K is a value necessary to calculate the total movement angle TMA of the tip portion 153.

In an embodiment, the calculating of the first value (S131) may include obtaining a first image i1 when the tip portion 153 is at a first angle a, and a second image i2 when the tip portion 153 is at a second angle b, and measuring a difference $\Delta\theta$ between the first angle a and the second angle b, and a position difference $\Delta X$ between the first image i1 and the second image i2. The first angle a and the second angle b are two different arbitrary angles for calculating the first value K, and may refer to angles between the tip portion 153 and the insertion unit 151, which are formed as the bending portion 152 is bent.

Referring to FIG. 4, the first image i1 may be obtained with the image sensor 153a when the tip portion 153 is caused to be at the first angle a by manipulating the bending portion 152. Here, the coordinates of an arbitrary point X inside the body on the image may be (xa, ya). The method of designating the arbitrary point X is not particularly limited. In an embodiment, the arbitrary point X may be the position of a blood vessel having a particular shape inside the body found on the image.

Again, the second image i2 may be obtained with the image sensor 153a when the tip portion 153 is caused to be at the second angle b by manipulating the bending portion 152. Here, the coordinates of an arbitrary point X inside the body on the image may be (xb, yb).

The coordinate difference between the first image i1 and the second image i2 may be expressed as $(\Delta x, \Delta y)$, which is the difference between the coordinates of the arbitrary point X on the images. Here, $\Delta x = xb - xa$, and $\Delta y = yb - ya$. By using this, the position difference $\Delta X$ between the first image i1 and the second image i2 is expressed as [Equation 1] above.

By applying the difference $\Delta\theta$ between the first angle a and the second angle b and the position difference $\Delta X$ between the first image i1 and the second image i2 to [Equation 2] above, the first value K may be calculated.

The endoscopic device 100 may calculate the total movement angle TMA of the tip portion based on the on-image lesion position information P and the first value K (S132). The total movement angle TMA refers to a total angle by which the tip portion 153 needs to move to move the coordinates $(x_1, y_1)$ of the center of the lesion LE to the center coordinates (0, 0) on the image. Referring to FIG. 3, the length Q from the center of the lesion LE to the center of the image is as shown in [Equation 3] above.

The total movement angle TMA may be calculated by applying the length Q from the center of the lesion LE to the center of the image, and the calculated first value K to [Equation 4] above.

The endoscopic device 100 may calculate the target angle ag for the current time by considering the movement angular velocity as and the control period cp of the tip portion 153 (S133). The movement angular velocity as and the control period cp of the tip portion 153 may be set in advance.

In an embodiment, the target angle ag for the current time may be calculated through a polynomial trajectory. As a specific example, the tip portion 153 may be set to move by the total movement angle TMA at a constant velocity. The preset movement angular velocity as of the tip portion 153 may be 30°/s, and the preset control period cp may be 2 ms. In this case, the target angle ag for the current time may be 30°/s × 2ms = 0.2 °. However, this is only an example for description and the disclosure is not limited thereto.

In another embodiment, the target angle ag for the current time may be calculated through a Bezier curve trajectory. That is, the method of calculating the target angle ag for the current time is not limited to the polynomial trajectory. In other words, the target angle ag for the current time may be calculated through various trajectories that may occur to one of skill in the art.

The endoscopic device 100 may move the tip portion 153 by the target angle ag for the current time (S134). The endoscopic device 100 may calculate a force necessary to move the tip portion 153 by the target angle ag for the current time, by considering the dynamic characteristics of the bending portion 152. The endoscopic device 100 may deliver information about the calculated force to the driving unit 130 to move the tip portion 153 by the target angle ag for the current time.

The identifying of the lesion (S120) and the controlling of the tip portion (S130) may be repeatedly performed until the lesion LE is located in the first area. Accordingly, the tip portion 153 may track the lesion LE, and the lesion LE may be continuously output on the display unit 110.

FIG. 9 is a flowchart of a method M2 of controlling an endoscopic device, according to another embodiment.

Referring to FIG. 9, the method M2 of controlling an endoscopic device may include obtaining an image inside a body from the image sensor 153a (S210), identifying a plurality of lesions LE from the image based on the pre-trained model 200 (S220), outputting on-image lesion position information P for the identified plurality of lesions LE (S230), selecting a target lesion T from among the plurality of lesions LE in response to a user input on the manipulation unit 140 (S240), and controlling the tip portion 153 to track one target lesion T from among the plurality of lesions LE (S250). Among the operations, the obtaining of the image (S210), the outputting of the on-image position information (S230), and the controlling of the tip portion (S250) are the same as or similar to those described above regarding the method M1 of controlling an endoscopic device, and thus, detailed descriptions thereof will be omitted and the differences therebetween will be mainly described.

The endoscopic device 100 may identify the plurality of lesions LE from the image based on the pre-trained model 200 (S220). For example, referring to FIG. 6, the first lesion LE1, the second lesion LE2, and the third lesion LE3 may be identified in the image obtained from the image sensor 153a.

In the outputting of the on-image lesion position information P (S230), the pre-trained model 200 may output bounding boxes surrounding the first lesion LE1, the second lesion LE2, and the third lesion LE3, respectively.

In response to a user input on the manipulation unit 140, the target lesion T may be selected from among the plurality of lesions LE (S240). In detail, in response to a user input, a lesion LE designated from among the plurality of lesions LE may be changed, and the designated lesion LE may be selected as the target lesion T. The user may select the target lesion T from among the plurality of lesions LE by using the selection button (not shown) provided on the manipulation unit 140.

In an embodiment, the user input may include at least one of a single input, an input for a threshold time or longer, and a double input within the threshold time. For example, the single input may be a command to change a lesion LE designated from among the plurality of lesions LE. In addition, the double input within the threshold time may be a command to select the designated lesion LE as the target lesion T. However, this is only an example and the disclosure is not limited thereto.

In an embodiment, as illustrated in FIG. 6, the designated lesion LE may be specified by the bounding box B having a different thickness. A detailed description with the above example is as follows. When the user presses the selection button once, the designated lesion LE may be changed from the first lesion LE1 to the second lesion LE2. When the user presses the selection button one more time, the designated lesion LE may change from the second lesion LE2 to the third lesion LE3. At this time, when the user presses the selection button twice within the threshold time, the designated third lesion LE3 may be selected as the target lesion T.

The practicality of the endoscopic device 100 may be improved by, when a plurality of lesions LE are identified in one image, tracking the target lesion T selected from among the plurality of lesions LE.

The disclosure is described above with reference to the embodiments illustrated in the drawings, but the embodiments are only examples. Those of skill in the art will understand that various modifications and other equivalent embodiments may be derived from the embodiments. Therefore, the true technical protection scope of the disclosure should be determined by the appended claims.

The particular technological details described in the embodiments are illustrative examples, and are not intended to limit the technological scope of the embodiments. In order to briefly and clearly describe the disclosure, descriptions of general techniques and configurations in the art may be omitted. Furthermore, line connections or connection members between elements depicted in the drawings represent functional connections and/or physical or circuit connections by way of example, and in actual applications, they may be replaced or embodied with various suitable additional functional connections, physical connections, or circuit connections. In addition, no item or component is essential to the practice of the disclosure unless the item or component is specifically described as being "essential" or "critical".

As used herein, the term "the" and other demonstratives similar thereto may include a singular form and plural forms, unless specifically limited. Furthermore, recitation of ranges of values herein are merely intended to serve as a shorthand method of referring individually to each separate value falling within the range, unless otherwise indicated herein, and each separate value is incorporated into the specification as if it were individually recited herein. In addition, the operations of all methods described herein may be performed in any suitable order unless otherwise indicated herein or otherwise clearly contradicted by context. The embodiments are not limited to the described order of the operations. The use of any and all examples, or exemplary language (e.g., "and the like") provided herein, is intended merely to better illuminate embodiments and does not pose a limitation on the scope of the embodiments unless otherwise claimed. In addition, those of skill in the art will understand that various modifications, combinations, and adaptations may be configured according to design conditions and factors without departing from the following claims and equivalents thereof.

[Explanation of Reference Numerals]

| | | | |
|---|---|---|---|
| 1: | Endoscopic device | | |
| 110: | Display unit | 120: | Control unit |
| 130: | Driving unit | 140: | Manipulation unit |
| 150: | Scope | 151: | Insertion unit |
| 152: | Bending unit | 153: | Tip portion |
| 153a: | Image sensor | 153b: | Lens |
| 153c: | Light | | |
| 200: | Pre-trained model | | |
| M1, M2: | Method of controlling anendoscopic device | | |
| LE: | Lesion | | |
| B: | Bounding box | | |
| K: | First value | | |
| P: | On-image lesion position information | | |

**Claims**

1. A method of controlling an endoscopic device, the method comprising:

   obtaining an image inside a body from an image sensor;
   identifying a lesion from the image based on a pre-trained model; and
   controlling a tip portion of the endoscopic device to track the lesion.

2. The method of claim 1, wherein an angle of view of the image sensor comprises a first area, and
   the controlling of the tip portion comprises controlling the tip portion such that the lesion is located within the first area.

3. The method of claim 1, wherein the identifying of the lesion comprises calculating on-image lesion position information indicating a position of the lesion on the image.

4. The method of claim 1, wherein the controlling of the tip portion comprises calculating a first value defined as a ratio of a difference in a position on the image to a change in an angle of the tip portion.

5. A method of controlling an endoscopic device, the method comprising:

   obtaining an image inside a body from an image sensor;
   identifying a plurality of lesions from the image based on a pre-trained model; and
   controlling a tip portion of the endoscopic device to track one target lesion from among the plurality of lesions.

6. An endoscopic device comprising:

   a tip portion comprising an image sensor; and
   a control unit configured to obtain an image inside a body from the image sensor, identify a lesion from the image based on a pre-trained model, and control the tip portion to track the lesion.

7. The endoscopic device of claim 6, wherein an angle of view of the image sensor comprises a first area, and
   the control unit is further configured to control the tip portion such that the lesion is located within the first area.

8. The endoscopic device of claim 7, wherein the first area comprises a center of the angle of view.

9. The endoscopic device of claim 6, wherein the control unit is further configured to determine on-image lesion position information indicating a position of the lesion on the image.

10. The endoscopic device of claim 6, wherein the control unit is further configured to calculate a first value defined as a ratio of a difference in a position on the image to a change in an angle of the tip portion.

11. The endoscopic device of claim 10, wherein the control unit is further configured to obtain a first image when the tip portion is at a first angle and obtain a second image when the tip portion is at a second angle, and measure a difference between the first angle and the second angle, and a position difference between the first image and the second image.

12. The endoscopic device of claim 10, wherein the control unit is further configured to calculate a total movement angle of the tip portion based on the first value.

13. An endoscopic device comprising:

a tip portion comprising an image sensor; and
a control unit configured to obtain an image inside a body from the image sensor, identify a plurality of lesions from the image based on a pre-trained model, and control the tip portion to track one target lesion from among the plurality of lesions.

14. The endoscopic device of claim 13, further comprising:

a display unit configured to output the image; and
a manipulation unit configured to receive an input from a user,
wherein the control unit is further configured to output, on the display unit, on-image lesion position information for the identified plurality of lesions, and
the target lesion is selected from among the plurality of lesions in response to a user input on the manipulation unit.

15. The endoscopic device of claim 14, wherein the user input comprises at least one of a single input, an input for a threshold time or longer, and a double input within the threshold time.

# FIG. 1

# FIG. 2

FIG. 3

# FIG. 4

FIG. 5

# FIG. 6

# FIG. 7

M1

```
        ┌─────────────┐
        │    START    │
        └──────┬──────┘
               │
               ▼
┌──────────────────────────────────┐
│   OBTAIN IMAGE INSIDE BODY        │──S110
│   FROM IMAGE SENSOR               │
└──────────────┬───────────────────┘
               │
               ▼
┌──────────────────────────────────┐
│   IDENTIFY LESION FROM IMAGE      │──S120
│   BASED ON PRE-TRAINED MODEL      │
└──────────────┬───────────────────┘
               │
               ▼
┌──────────────────────────────────┐
│ CONTROL TIP PORTION TO TRACK LESION │──S130
└──────────────┬───────────────────┘
               │
               ▼
        ┌─────────────┐
        │     END     │
        └─────────────┘
```

# FIG. 8

```
        ┌─────────────┐
        │    START    │
        └─────────────┘
               │
               ▼
┌───────────────────────────────────────┐
│         CALCULATE FIRST VALUE          │──S131
└───────────────────────────────────────┘
               │
               ▼
┌───────────────────────────────────────┐
│   CALCULATE TOTAL MOVEMENT ANGLE OF    │
│   TIP PORTION BASED ON ON-IMAGE LESION │──S132
│   POSITION INFORMATION AND FIRST VALUE │
└───────────────────────────────────────┘
               │
               ▼
┌───────────────────────────────────────┐
│   CALCULATE TARGET ANGLE FOR CURRENT   │
│   TIME BY CONSIDERING MOVEMENT ANGULAR │──S133
│ VELOCITY AND CONTROL PERIOD OF TIP PORTION │
└───────────────────────────────────────┘
               │
               ▼
┌───────────────────────────────────────┐
│      MOVE TIP PORTION BY TARGET ANGLE  │──S134
└───────────────────────────────────────┘
               │
               ▼
        ┌─────────────┐
        │     END     │
        └─────────────┘
```

# FIG. 9

M2

```
        ┌──────────────┐
        │    START     │
        └──────┬───────┘
               │
               ▼
┌──────────────────────────────────┐
│      OBTAIN IMAGE INSIDE BODY     │── S210
│        FROM IMAGE SENSOR          │
└──────────────┬───────────────────┘
               │
               ▼
┌──────────────────────────────────┐
│   IDENTIFY PLURALITY OF LESIONS   │── S220
│   FROM IMAGE BASED ON             │
│   PRE-TRAINED MODEL               │
└──────────────┬───────────────────┘
               │
               ▼
┌──────────────────────────────────┐
│   OUTPUT ON-IMAGE LESION POSITION │── S230
│   INFORMATION FOR PLURALITY       │
│   OF LESIONS                      │
└──────────────┬───────────────────┘
               │
               ▼
┌──────────────────────────────────┐
│       SELECT TARGET LESION        │── S240
└──────────────┬───────────────────┘
               │
               ▼
┌──────────────────────────────────┐
│      CONTROL TIP PORTION TO       │── S250
│      TRACK TARGET LESION          │
└──────────────┬───────────────────┘
               │
               ▼
        ┌──────────────┐
        │     END      │
        └──────────────┘
```

Europäisches
Patentamt

European
Patent Office

Office européen
des brevets

# EUROPEAN SEARCH REPORT

**Application Number**

EP 24 15 9271

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/296281 A1 (YEUNG CHUNG-KWONG [CN] ET AL) 18 October 2018 (2018-10-18) * paragraphs [0010], [0041], [0046] - [0048], [0050] * | 1-15 | INV. A61B1/00 A61B1/005 |
| X | US 2021/059607 A1 (GORMLEY WILLIAM B [US] ET AL) 4 March 2021 (2021-03-04) * paragraphs [0079], [0094] * | 6 | |

TECHNICAL FIELDS SEARCHED (IPC)

A61B
G06T

The present search report has been drawn up for all claims

| Place of search | Date of completion of the search | Examiner |
|---|---|---|
| Munich | 31 July 2024 | Hemb, Björn |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 15 9271

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2024

| Patent document cited in search report | Publication date | Patent family member(s) | | Publication date |
|---|---|---|---|---|
| US 2018296281 A1 | 18-10-2018 | CN | 108685560 A | 23-10-2018 |
| | | US | 2018296281 A1 | 18-10-2018 |
| | | WO | 2018188466 A1 | 18-10-2018 |
| US 2021059607 A1 | 04-03-2021 | EP | 4213757 A1 | 26-07-2023 |
| | | JP | 2023545637 A | 31-10-2023 |
| | | US | 2021059607 A1 | 04-03-2021 |
| | | WO | 2020191269 A1 | 24-09-2020 |
| | | WO | 2022061313 A1 | 24-03-2022 |

EPO FORM P0459

For more details about this annex : see Official Journal of the European Patent Office, No. 12/82